(19) Europäisches Patentamt European Patent Office Office européen des brevets

(11) **EP 4 637 533 B1**

(12) **EUROPEAN PATENT SPECIFICATION**

(45) Date of publication and mention of the grant of the patent:
**25.03.2026 Bulletin 2026/13**

(21) Application number: **23821675.8**

(22) Date of filing: **13.12.2023**

(51) International Patent Classification (IPC):
*A61B 5/022* (2006.01)    *A61B 5/00* (2006.01)
*A61B 5/021* (2006.01)

(52) Cooperative Patent Classification (CPC):
**A61B 5/022; A61B 5/021; A61B 5/02108;
A61B 5/02225; A61B 5/6843; A61B 5/7203;
A61B 5/7207; A61B 5/7221;** A61B 2560/0223

(86) International application number:
**PCT/EP2023/085470**

(87) International publication number:
**WO 2024/132735 (27.06.2024 Gazette 2024/26)**

(54) **DETECTING AN ARTEFACT IN DATA**

ERKENNUNG EINES ARTEFAKTS IN DATEN

DÉTECTION D'UN ARTEFACT DANS DES DONNÉES

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **22.12.2022 EP 22215921**

(43) Date of publication of application:
**29.10.2025 Bulletin 2025/44**

(73) Proprietor: **Koninklijke Philips N.V.
5656 AG Eindhoven (NL)**

(72) Inventors:
• **DUINEVELD, Paulus Cornelis**
  **5656 AG Eindhoven (NL)**
• **VAN DE VEEN, Egbert**
  **5656 AG Eindhoven (NL)**
• **LOUWSMA, Hendrik Klaas**
  **5656 AG Eindhoven (NL)**
• **AMINE EL SAYED, Achraf**
  **5656 AG Eindhoven (NL)**
• **SCHIPPER, Niels**
  **5656AG Eindhoven (NL)**
• **YAU, Kam Hing**
  **5656AG Eindhoven (NL)**
• **WESSELS, Arnoldus Cornelis**
  **5656AG Eindhoven (NL)**
• **VAN DER VLIS, Peter Hans**
  **5656AG Eindhoven (NL)**
• **TAMMINGA, Stephanus Jacob Gerardus**
  **5656AG Eindhoven (NL)**

(74) Representative: **Philips Intellectual Property &
Standards
High Tech Campus 52
5656 AG Eindhoven (NL)**

(56) References cited:
**EP-A1- 0 563 425      US-A1- 2012 245 478
US-A1- 2021 121 073**

## Description

FIELD OF THE INVENTION

The invention relates to the general field of blood pressure measurements and, more particularly, to detecting an artefact in data acquired using a blood pressure cuff.

BACKGROUND OF THE INVENTION

US2012245478A1 is directed to systems and methods for detecting cuff slippage in a blood pressure monitoring device.

US2021121073A1 relates to an apparatus and a method of operating the apparatus for use with a wearable cuff in determining blood pressure and/or pulse rate.

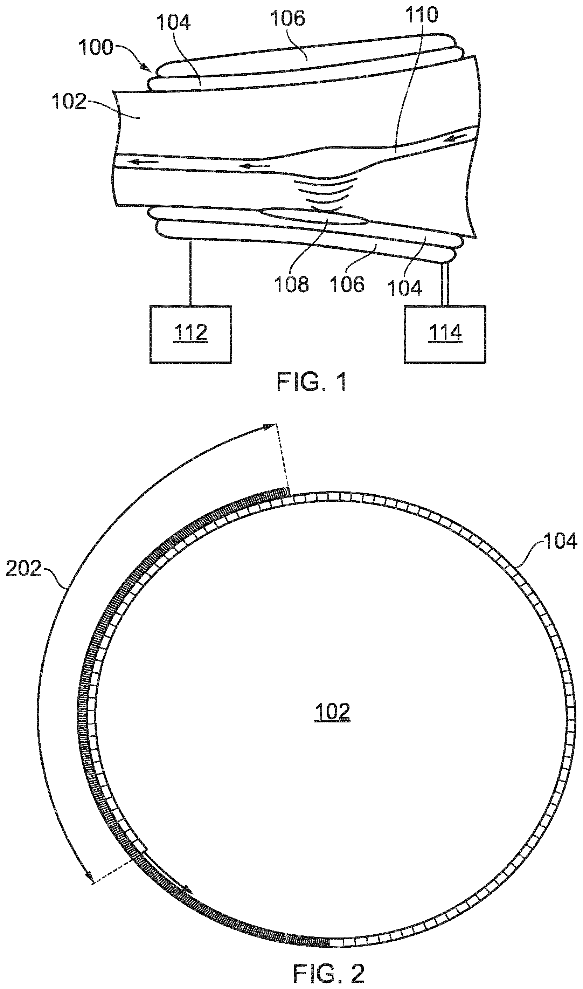

A subject's blood pressure can be measured using non-invasive blood pressure measurement technology, which may be implemented into a cuff. In an example, a cuff is configured to be wrapped around an arm of a subject in order to measure the blood pressure of the subject. Fig. 1 is a sectional illustration of an example of a blood pressure cuff 100 on an arm 102 of a subject. The blood pressure cuff 100 includes a shell 104 surrounded by an inflatable cuff portion 106. A sensor pad 108 on the shell 104 is configured to engage the subject's skin during use, and to measure a pressure wave resulting from the subject's pulse as it pumps blood through an artery 110 (e.g., the brachial artery) of the subject.

The pulse waves in the artery transmit through the subject's tissue and skin, causing movement of the skin. This skin movement causes a compression of the sensor pad 108 which can be registered by a pressure sensor 112 as a change in pressure.

An actuator 114 (e.g., an air pump) may be provided to deliver (e.g., pump) a gas such as air into the inflatable cuff portion. As the inflatable cuff portion 106 inflates, the arm tissue becomes compressed, so the brachial artery 110 can be closed.

Fig. 2 is a schematic end view illustration of shell 104 of the blood pressure cuff 100 on the arm 102 of the subject. During inflation of the inflatable cuff portion 106, the pressure in the inflatable cuff portion and the pressure measured by the pressure sensor 112 increase. Due to the increasing pressure on the arm, the arm is compressed and therefore its diameter reduces. For the shell 104 of the cuff to fully enclose the arm, the diameter of the shell also needs to reduce and, therefore, an overlap region 202 of the shell is provided. As the pressure in the inflatable cuff portion 106 increased, the amount of overlap of the shell 104 in the overlap region 202 increases. Ideally, the overlapping parts of the shell 104 in the overlap region 202 can easily slide with respect one another and therefore the overlapping parts typically have a relatively low coefficient of friction.

In some cases, the pulse from the brachial artery may be large enough to interrupt the sliding of the overlap region 202 of the shell 104 and the shell may be caused to temporarily stick before it starts to slide again. This behaviour may be referred to as a "stick-slip" event, and may be responsible for a sudden and temporary artificial increase in the tissue pressure. Such increases may be referred to as artefacts in the data. If the brachial artery pulse is very severe, in combination with a low friction force, the overlap region 202 of the shell 104 may, after stopping sliding in one direction, be caused to open slightly in the other direction before continuing to slide in the original direction. Such disturbances can cause errors in determining hemodynamic parameters, such as Systolic Arterial Blood Pressure (SAP), Mean Arterial Pressure (MAP), Diastolic Arterial Blood Pressure (DAP), Stroke Volume (SV), heart rate, or the like.

To complicate matters, temporary increases in tissue pressure may be caused by other, natural events, so there is a need to not only identify artefacts in the data, but also to distinguish artefacts resulting from natural events from artefacts occurring as a result of stick-slip events or other non-physiological events having an influence on the hardware, such as motion artefacts, sensor defects, touching of supply lines to the device and so on.

SUMMARY OF THE INVENTION

It would be desirable to be able to recognise an artefact in a set of data obtained using a blood pressure cuff, and optionally determining whether or not the artefact is a result of a stick-slip event. The inventors of the present disclosure have recognised that, by analysing the obtained data in a particular way, such a determination may be made. In this way, appropriate action may be taken if it is determined that one or more stick-slip events (or other artefact-causing events) have occurred.

According to a first specific aspect, there is provided a computer-implemented method for detecting an artefact in data acquired using a blood pressure measurement device, the method comprising receiving applied pressure data, the applied pressure data comprising data indicative of a pressure applied to a body part of a subject whose blood pressure is to be measured; receiving tissue pressure data, the tissue pressure data indicative of a tissue pressure applied by the tissue of the subject on a sensor pad of the blood pressure measurement device; determining the first derivative of the applied pressure data; identifying an occurrence in the first derivative of the applied pressure data where an amplitude of the first derivative of the applied pressure data meets a first defined threshold condition; and responsive to determining that, within a defined duration of the occurrence, the tissue pressure meets a second defined threshold condition, determining that there exists an artefact in the tissue pressure data.

In some embodiments, the method may further comprise generating, for delivery to a recipient device, a notification of the presence of the artefact in the tissue pressure data. The applied pressure data may be received from an applied pressure sensor. The tissue pressure data may be received from a sensor pad comprised by the blood pressure measurement device. The tissue pressure data may be received from a sensor pad which is arranged between a compression portion of the blood pressure measurement device (configured to apply pressure to the body part of the device) and the body part itself during operation of the device. The sensor pad is different/separate to the applied pressure sensor. Thus, the applied pressure data and the tissue pressure data may be independently sensed signals.

The method may further comprise determining a correction to be applied to the tissue pressure data to remove the artefact; and applying the correction to the tissue pressure data.

In some embodiments, determining the correction may comprise interpolating the first derivative of the tissue pressure data over a defined interpolation time period.

The method may, in some embodiments, further comprise responsive to determining that the tissue pressure meets the second defined threshold condition not within the defined duration of the occurrence, determining that the tissue pressure data includes an event indicative of a hemodynamic effect.

The hemodynamic effect may, for example, comprise at least one of a dicrotic notch event and a heart contractility event.

The method may further comprise responsive to determining that the artefact is one of a plurality of artefacts identified in the tissue pressure data, and that the number of artefacts in the plurality of artefacts exceeds a defined threshold number, generating, for delivery to a recipient device, a notification that action should be taken with regard to the blood pressure measurement device.

In some embodiments, the method may further comprise applying a data flattening operation to at least one of the applied pressure data and the tissue pressure data.

In some embodiments, determining the first derivative of the applied pressure data comprises determining the negative first derivative of the applied pressure data. In such cases, identifying an occurrence where an amplitude of the first derivative of the applied pressure data meets a first defined threshold condition may comprise identifying an occurrence where the amplitude of the first derivative is greater than 0.

The defined duration may comprise a duration of 100 ms before or after the occurrence.

In some embodiments, the second defined threshold condition may be met when the amplitude of the tissue pressure increases above a defined threshold pressure within the defined duration of the occurrence, the defined threshold pressure being between 0.05 and 50 mmHg.

In some embodiments, the method further comprises determining one or more hemodynamic parameters of the subject based on processing of the tissue pressure data, for example based on application of a parameter quantification algorithm to the tissue pressure data.

In some embodiments, the method may further comprise, following the determining that there exists an artefact in the tissue pressure data, determining whether the artefact is a stick-slip artefact. Options relating to this process will now be outlined below. It is noted that the below-outlined procedure for determining whether an artefact is a stick-slip artefact can be applied with advantageous effect regardless of the particular method by which the presence (or suspected presence) of an artefact in the signal is initially detected. In other words, the below method/procedure for determining/classifying whether an artefact is a stick-slip artefact is a separate inventive concept and may be provided as an independent aspect of the invention. Thus notwithstanding, this concept is also compatible with any of the embodiments or features described above, and may be combined with any one or more of these to further advantageous effect.

In some embodiments, the determining whether an artefact is a stick-slip artefact may comprise retrieving a pre-defined artefact model which models an expected magnitude of a stick-slip artefact in tissue pressure data received from a sensor pad as a function of position of the artefact in the data. The position of the artefact in the data may mean a position in time of the artefact relative to one or more other features in the tissue pressure signal, for example relative to a pulse wave in the tissue pressure signal, for example relative to a start of a temporally closest pulse wave which temporally precedes the artefact in the signal. For example, the position may be defined by a time difference between a start (e.g. a foot) of a pulse wave in the signal immediately preceding the artefact and a start of the artefact. For reasons discussed later, these time points may in general be assumed to correspond to the start of the stick phase of the stick-slip event with which a stick-slip artefact is associated and the start of the slip phase of the stick-slip event, wherein it is during the slip phase that the signal artefact arises.

The determining whether the artefact is a stick slip artefact may further comprise: determining a position of the detected artefact in the tissue pressure data, e.g. relative to at least one pulse wave in the data as discussed above, and determining a true magnitude of the artefact in the tissue pressure data; computing an expected magnitude of the artefact in the tissue pressure data if the artefact were a stick slip artefact based on use of the model and the determined position of the detected artefact; comparing the expected magnitude of the artefact with the true magnitude of the artefact; and classifying the artefact as a stick-slip artefact only if the true magnitude falls within a defined proximity range of the expected magnitude. The magnitude of the artefact or suspected artefact may mean the height of the artefact in the tissue pressure signal, i.e. the height in pressure terms.

More concretely, in some embodiments, the artefact model models an expected magnitude, $\Delta p_{j,theory}$, of a stick-slip artefact in tissue pressure data received from a sensor pad according to the equation:

$$\Delta p_{j,theory} \approx \beta\left(t_{slip} - t_{stick}\right)$$

where the tissue pressure data includes a baseline slope component associated with the increasing applied pressure and an oscillation component associated with arterial pressure oscillations, and where $\beta$ is the gradient of the baseline slope component of the tissue pressure data, $t_{stick}$ is a time point of a start of a stick phase of the stick-slip artefact, and $t_{slip}$ is the time point of the start of a slip phase (or stop of the stick phase) of the stick slip artefact. The time point, $t_{stick}$ may be determined/estimated or defined as the time point coinciding with the start point (or foot) of a pulse wave in the tissue pressure signal immediately preceding the artefact, and the time point $t_{slip}$ may be determined as the time point of the start of the detected artefact.

In some embodiments, the artefact is classified as a confirmed stick-slip artefact only if the true magnitude, $\Delta p_j$, of the detected artefact in the tissue pressure signal from the sensor pad, i.e. height of the artefact in the tissue pressure signal, meets the condition:

$$0.5\,\Delta p_{j,theory} \leq \Delta p_j \leq 1.5\Delta p_{j,theory}$$

where $\Delta p_{j,theory}$ is the expected magnitude of the artefact according to the model. In other words, the defined proximity range may be defined by $0.5\,\Delta p_{j,theory} \leq \Delta p_j \leq 1.5\Delta p_{j,theory}$.

According to a further specific aspect, there is provided an apparatus for detecting an artefact in data acquired using a blood pressure measurement device, the apparatus comprising a processor configured to perform steps of the methods disclosed herein.

According to a further specific aspect, there is provided a system for detecting an artefact in data acquired using a blood pressure measurement device, the system comprising a blood pressure measurement device comprising a sensor pad configured to measure a pressure applied on the sensor pad by tissue of a body part of a subject wearing the blood pressure measurement device; and a compression portion configured to apply pressure to the body part of the subject. The system comprises an applied pressure sensor configured to measure the pressure applied to the body part of the subject by the compression portion; and a processor configured to receive, from the applied pressure sensor, applied pressure data indicative of the pressure applied to the body part of the subject; receive, from the sensor pad, tissue pressure data indicative of a pressure applied by the tissue of the body part of the subject on the sensor pad; determine the first derivative of the applied pressure data; identify an occurrence in the first derivative of the applied pressure data where an amplitude of the first derivative of the applied pressure data meets a first defined threshold condition; and responsive to determining that, within a defined duration of the occurrence, the tissue pressure meets a second defined threshold condition, determine that there exists an artefact in the tissue pressure data.

The sensor pad may be arranged between the compression portion of the device and the subject's body part during use of the device. The sensor pad is a separate/independent sensing device to the applied pressure sensor. Thus the applied pressure data and the tissue pressure data may be independently sensed signals.

The system may further comprise a user interface configured to present, to a recipient, a notification indicating the presence of the artefact in the tissue pressure data.

According to a further specific aspect, there is provided a computer program product comprising a non-transitory computer-readable medium, the computer-readable medium having computer-readable code embodied therein, the computer-readable code being configured such that, on execution by a suitable computer or processor, the computer or processor is caused to perform steps of the methods disclosed herein.

These and other aspects will be apparent from and elucidated with reference to the embodiment(s) described hereinafter.

## BRIEF DESCRIPTION OF THE DRAWINGS

Exemplary embodiments will now be described, by way of example only, with reference to the following drawings, in which:

Fig. 1 is an illustration of an example of a blood pressure cuff on a body part of a subject;
Fig. 2 is a schematic illustration of a shell of a blood pressure cuff around a body part of a subject;
Fig. 3 is a flowchart of an example of a method for detecting an artefact in data acquired using a blood pressure measurement device;
Fig. 4 is a flowchart of a further example of a method for detecting an artefact in data acquired using a blood pressure measurement device;
Fig. 5 is a set of graphs showing a set of data acquired using a blood pressure measurement device;
Fig. 6 is a set of graphs showing a further set of data acquired using a blood pressure measurement device;
Fig. 7 is a set of graphs showing a further set of data acquired using a blood pressure measurement device;
Fig. 8 is a set of graphs showing a further set of data acquired using a blood pressure measurement device;

Fig. 9 is a schematic illustration of an example of an apparatus for detecting an artefact in data acquired using a blood pressure measurement device;

Fig. 10 is a schematic illustration of an example of a system for detecting an artefact in data acquired using a blood pressure measurement device;

Fig. 11 is a schematic illustration of an example of a processor in communication with a computer-readable medium;

Fig. 12 shows an in-vivo recording of applied pressure and tissue pressure signals; and

Fig. 13 shows a sensor pad pressure signal including several heart-beat cycles, each including a stick-slip event.

## DETAILED DESCRIPTION OF EMBODIMENTS

The inventors of the present disclosure have recognised that an artefact in data acquired using a blood pressure measurement device can be attributed to a stick-slip event or a heart contractility event by analysing the data and, in particular, by looking at a combination of data relating to a pressure applied to the body part of the subject while the subject's blood pressure is measured and data relating to a pressure applied by the body part on a pressure sensor.

Various examples will now be described, with reference to a blood pressure measurement device, such as the device shown in Figs. 1 and 2. As described with reference to Fig. 1, such a device may include a shell 104 that is caused to close around a body part (e.g., an arm) of a subject by a compression portion (also referred to as a pressurisation means or actuator). As discussed in greater detail below, the compression portion may include one or more straps wrapped around the shell 104 which, apply a greater pressure on the subject's body part as they are tightened. **In** other examples, such as the example shown in Fig. 1, the compression portion may comprise an inflatable cuff capable of receiving gas (e.g., air), such that the inflatable cuff portion applies a greater pressure on the subject's body part as the pressure of gas in the inflatable cuff portion is increased. The pressure applied to the body part by the compression portion may be measured using a sensor referred to herein as an applied pressure sensor.

It has been recognised that a stick-slip event can cause a sudden increase in tissue pressure (i.e., the pressure applied by the tissue of the body part of the subject on a sensor pad of a blood pressure measurement device). The increase in tissue pressure causes a sudden increase in pressure applied to the body part by the blood pressure measurement device and, a sudden tissue compression and decrease in the diameter of the body part. As the diameter of the body part decreases, a pressure measured using the applied pressure sensor can be seen to experience a sudden decrease, since the reduced diameter of the body part provides more space for the compression portion to expand into.

It has also been recognised that, as the subject's heart contracts to pump blood through the arteries of their body, leading to a sudden increase in the tissue pressure measured by the sensor pad, the diameter of the body part increases slightly, thereby increasing the pressure applied to the body part by the compression portion (i.e., the applied pressure). Thus, it is possible to determine whether a sudden change in tissue pressure is likely to be caused by a stick-slip event or by a naturally occurring haemodynamic event.

More generally, it is the further recognition of the inventors that the same principle can also be used to detect artefacts caused by a variety of other types of non-physiological event. As a principle of general application, it can be stated that a truly physiologically caused fluctuation in tissue pressure is unlikely to be accompanied by a concurrent fluctuation in the rate of applied pressure being exerted by the pressure applicator. The concurrent occurrence of these two conditions is an indicator of a non-physiological influence on the tissue pressure sensor reading, regardless of the particular cause. One particular and most common cause of artefacts which will be picked up by this detection mechanism is stick slip artefacts. However, other events which might cause a similar such confluence of conditions include certain types of motion artefact (e.g. a user moving or tugging at the pressure applicator), a more global slippage of the attachment of the pressure applicator to the body part, a defect in one of the sensors, a touching or hitting of the pressure applicator accidentally by a user or caregiver, a slipping of the pressure applicator down the body part and so on. Each of these would manifest as a fluctuation in both the rate of change of the applied pressure and in the measured tissue pressure.

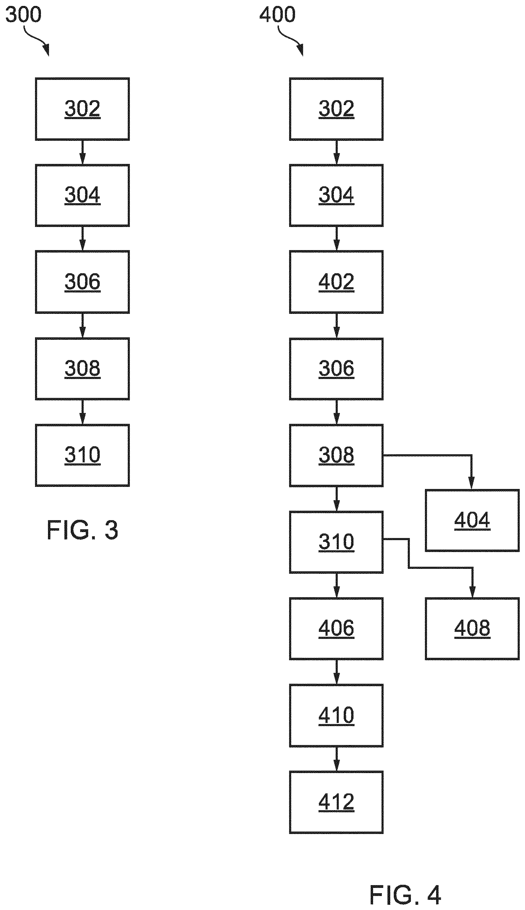

According to a first aspect, a method is provided. Referring to the drawings, Fig. 3 is a flowchart of an example of a method 300 for detecting an artefact in data acquired using a blood pressure measurement device. The method 300 may comprise a computer implemented method and, as such, steps of the method may be performed using one or more processors or processing circuitry. The method 300 comprises, at step 302, receiving applied pressure data. The applied pressure data comprises data indicative of a pressure applied to a body part of a subject whose blood pressure is to be measured. As noted above, the applied pressure may comprise the pressure applied by the compression portion of the blood pressure measurement device. For example, the applied pressure data may be indicative of a pressure of a gas in an inflatable cuff portion, or the pressure applied using some other pressurisation means. The applied pressure may be measured by an applied pressure sensor. By way of example, this may be a sensor arranged for sensing a pressure of a gas in an inflatable cuff portion or the pressure applied using some other pressurization means.

At step 304, the method 300 comprises receiving tissue pressure data. The tissue pressure data is indicative of a tissue pressure applied by the tissue of the subject on a sensor pad of the blood pressure measurement device. As discussed above, the sensor pad (e.g., the sensor pad 108 of Fig. 1) may be used to measure a pressure or a change in pressure within tissue of the body part of the subject. In an example, the sensor pad may comprise a flexible bag at least partially filled with a liquid which is caused to compress when the skin and tissue of the body part move. The sensor pad may be connected to a pressure sensor via a flexible tube which is also filled with a liquid (e.g., the same or similar liquid to the liquid in the sensor pad), and the pressure sensor acquires data relating to the tissue pressure.

Various methods of applying pressure to a body part of a subject and measuring the pressure applied and the tissue pressure are described in US patent number 8998817, European patent number 2953528 and International patent application number PCT/EP2022/075999.

The applied pressure data and/or the tissue pressure data may be measured and/or received in real time, or as close as possible to real time. For example, data may be sampled (i.e., data may be measured and/or received) with a frequency of at least 50 Hz, preferably with a frequency of at least 125 Hz, and more preferably with a frequency of at least 1 kHz.

The method 300 comprises, at step 306, determining the first derivative of the applied pressure data. Specifically, the first derivative of the applied pressure data is determined over time. In this way, sudden changes in the gradient of a data signal containing the applied pressure data can be more easily identified.

At step 308, the method 300 comprises identifying an occurrence in the first derivative of the applied pressure data where an amplitude of the first derivative of the applied pressure data meets a first defined threshold condition. The first defined threshold condition may, for example, comprise a threshold pressure value, above which the first defined threshold condition is met. In some embodiments, an average value or baseline value for the first derivative of the applied pressure data may be determined, and the first defined threshold condition may be met if the first derivative of the applied pressure data increases beyond a defined amount above the average or baseline level. Thus, the first defined threshold condition may be met if the first derivative of the applied pressure data increases by more than a defined absolute amount or a defined relative amount.

Once an occurrence has been identified where the first defined threshold condition is met, the data may be investigated further, to determine the nature of the occurrence. According to the present invention, the tissue pressure data is analysed over a period of time corresponding to the occurrence in the applied pressure data, to see whether the tissue pressure data meets a particular threshold condition. Thus, the method 300 comprises, at step 310, responsive to determining that, within a defined duration of the occurrence, the tissue pressure meets a second defined threshold condition, determining that the occurrence comprises an artefact in the tissue pressure data. In other words, if the tissue pressure data does meet the second defined threshold condition within the defined duration of the occurrence in the applied pressure data, then it may be determined that there exists an artefact in the tissue pressure data. As discussed in greater detail below, an artefact in the tissue pressure data may be indicative of a stick-slip event or other non-physiological event, and in response to detecting such an event, appropriate action may be taken, such as carrying out further investigations of the blood pressure measurement device.

The second defined threshold condition may be selected based on the blood pressure measurement device used and/or the subject. In some embodiments, the second defined threshold condition may be met when the amplitude of the tissue pressure increases above a defined threshold pressure within the defined duration of the occurrence. The defined threshold pressure may be between 0.05 mmHg and 50 mmHg. in other examples, the defined threshold pressure may be between 0.1 mmHg and 15 mmHg. and, in other examples, a different defined threshold pressure may be used.

An advantage of performing the method 300 is that a stick-slip event may be distinguished from some other (physiological) event, such as a haemodynamic event, that may also be apparent in the data.

Fig. 4 is a flowchart of a further example of a method 400 for detecting an artefact in data acquired using a blood pressure measurement device. The method 400 may comprise steps of the method 300 discussed above. In some embodiments, the method 400 may further comprise, at step 402, applying a data flattening operation to at least one of the applied pressure data and the tissue pressure data. A data flattening operation may, for example, be applied to remove a global pressure increase in the tissue pressure as a result of increasing applied pressure (e.g., as an inflatable cuff is inflated), such that the flattened data shows the response of the tissue pressure to the subject's heartbeat. A flattening operation may also be applied to the applied pressure data, and this can make it easier to interpret gradients in the applied pressure data and the tissue pressure data. Various techniques may be used to apply the data flattening operation. In some embodiments, the data may be filtered using a low pass frequency filter. In other embodiments, an average curve of the data (e.g., calculated using a moving average) may be subtracted from the data. In other embodiments, other signal filtering techniques may be used.

In embodiments where a flattening operation is applied to the data at step 402, then subsequent steps (e.g., step 306, 308 and 310) of the method 300 may be performed in respect of the flattened applied pressure data and/or the flattened tissue pressure data. In some embodiments, determining the first derivative of the applied pressure data may comprise determining the negative first derivative of the applied pressure data. In this way, changes in the applied pressure data will appear as peaks. In such examples, when analysing flattened data, identifying an occurrence (step 308) where an amplitude of the first derivative of the applied pressure data meets a first defined threshold condition may comprise identifying an occurrence where the amplitude of the first derivative is greater than 0. In other examples, a different first threshold condition may be applied. For example, when a flattening operation is not applied to the data, then the first defined threshold condition may be based on an average of the data.

Data shows that the first derivative of the applied pressure data (e.g., the pressure gradient) typically ranges from 0.8 mm Hg/s to 9 mm Hg/s and, for most people, it ranges from around 2 mm Hg/s to 3 mm Hg/s. The pressure gradient for the received tissue data (excluding the effect of the subject's heartbeat) is typically around 50% to 95% of the applied pressure gradient. The defined threshold condition used when a flattening operation is not applied to the data may be similar to when the flattening operation is applied, since the data that is removed as a result of the flattening operation has a generally constant gradient. Thus, the first defined threshold condition may be met if the change in gradient (i.e., the increase in the first derivative of the applied pressure data is greater than 0 mm Hg/s, 0.1 mm Hg/s, 0.2 mm Hg/s, 0.4 mm Hg/s, or the like.

In some examples, the first threshold condition may comprise a threshold amplitude of the first derivative of the applied pressure data of slightly higher, such as 1 mm Hg/s, 2 mm Hg/s or the like. In this way, it is possible to take account of variations in the data caused by or resulting from other factors, such as a pump used to inflate the inflatable cuff, for example. Another way of taking account of variations resulting from other factors is to base the first threshold condition on the duration that the first derivative of the applied pressure data is more than 80% of its peak value. For example, a peak in the data may be recognised as a possible stick-slip event if the duration that the first derivative of the applied pressure data is more than 80% of its peak value is less than 0.15 s, or more preferably less than 0.1 s. In examples where a flattening operation is applied to the tissue data, the second defined threshold condition may be met if the tissue pressure gradient (i.e., a first derivative of the tissue pressure) increased by more than 5 mm Hg/s or, more preferably, more than 20 mm Hg/s,

At step 310 of the method 300, the tissue pressure data is analysed to determine whether it meets a second defined threshold condition within a defined duration of the occurrence of a peak in the amplitude of the first derivative of the applied pressure data. If both the first defined threshold condition and the second defined threshold condition are met within the defined duration, then it is determined that an artefact exists in the tissue pressure data. However, at step 404, the method 400 makes a determination in the event that the second defined threshold condition is not met within the defined duration. Thus, at step 404, the method 400 may further comprise, responsive to determining that the tissue pressure meets the second defined threshold condition at a time not within the defined duration of the occurrence, determining that the tissue pressure data includes an event indicative of a hemodynamic effect. **In** other words, if a sudden change in pressure (e.g., a peak) occurs in the tissue data, but there is no sudden change (e.g., a peak) within the defined duration in the applied pressure data, then it can be determined that the change in tissue pressure is likely to have resulted from a haemodynamic effect, rather than a stick-slip event.

Examples of haemodynamic effects that might cause a change in pressure in the received pressure data include a dicrotic notch and heart contractility. Thus, the haemodynamic effect may comprise at least one of a dicrotic notch event and a heart contractility event. The dicrotic notch is the result of a short period where the flow of blood changes immediately before the aortic valve closes, and this can lead to a sudden change in tissue pressure. Thus, the dicrotic notch may cause a sudden increase in the tissue pressure, but no accompanying sudden increase (i.e., the threshold condition) in the applied pressure data is seen.

In some embodiments, the defined duration discussed herein may comprise a duration of 100 ms before or after the occurrence (i.e., occurrence in the first derivative of the applied pressure data where an amplitude of the first derivative of the cuff applied pressure data meets a first defined threshold condition). In other words, the tissue pressure data may be analysed in a 200 ms window centred on the time at which the occurrence occurred in the applied pressure data. In other embodiments, the defined duration may comprise a duration of 50 ms, 25 ms, 15 ms, 10 ms, 5 ms, 2 ms, or 1 ms before and/or after the occurrence. A different defined duration may alternatively be used.

Once the existence of an artefact in the tissue pressure data has been identified (step 310), appropriate action may be taken. In some examples, the method 400 may comprise, at step 406, generating, for delivery to a recipient device, a notification of the presence of the artefact in the tissue pressure data. The recipient device may, for example, comprise a computing device, a smart phone, a wearable device, a tablet computer, or the like, with an interface capable of communicating with, or notifying a recipient. In an example, a medical professional may be notified via the recipient device that an artefact in the tissue pressure data exists, and that this might be caused by a stick-slip event.

Stick-slip events themselves are not particularly problematic, as long as an occurrence in the data can be identified as a stick-slip event, rather than an anomaly in the subject's heartbeat. However, if multiple stick-slip events occur (e.g., within a defined timeframe, or while a subject's blood pressure is being taken), then this could be indicative of a fault in the blood pressure measurement device, or the way that the device has been attached to the subject. At step 408, the method 400 may further comprise responsive to determining that the artefact is one of a plurality of artefacts identified in the tissue pressure data, and that the number of artefacts in the plurality of artefacts exceeds a defined threshold number, generating, for delivery to a recipient device, a notification that action should be taken with regard to the blood pressure measurement device. In some embodiments, the notification to be delivered to the recipient device may indicate that the blood pressure measurement device (e.g., a blood pressure cuff) should be adjusted or refitted, or that the device should be replaced. If too many stick-slip events occur, it can be difficult to identify events occurring in the data that are caused by physiological problems, such as problems with the subject's heart.

In the event that a stick-slip event has been detected (i.e., it is determined at step 310 that an artefact exists in the tissue pressure data), then the tissue pressure data may, in some embodiments, be corrected or adjusted to take account of the stick-slip event and/or to remove the effect of the stick-slip event from the data. Thus, the method 400 may comprise, at step 410, determining a correction to be applied to the tissue pressure data to remove the artefact. At step 412, the method 400 may comprise applying the correction to the tissue pressure data. In some embodiments, determining the correction (step 410) may comprise interpolating the first derivative of the tissue pressure data over a defined interpolation time. The interpolation may, in some embodiments, be done over a time window beginning at, or within a short time (e.g., 0.1 seconds, 0.05 seconds or .01 seconds) of the point where the occurrence begins (e.g., where the negative first derivative of the flattened applied pressure data becomes positive). The interpolation involves interpolating the tissue pressure generated in a time window of between 0.001 seconds to 0.5 seconds or, more preferably, in time window of between 0.01 seconds to 0.1 seconds. In some embodiments, the interpolation may be made on the tissue pressure itself while, in other embodiments, the interpolation may be based on first and second derivatives of the tissue pressure, and/or on information relating to the heartbeats adjacent to the occurrence. The interpolation is continued up until the point at which the occurrence ends (e.g., where the negative first derivative of the flattened applied pressure data is between -0.3 mmHg/s and 0.3 mmHg/s, and, more preferably, between -0.05 mmHg/s and 0.1 mmHg/s.

In other embodiments, other types of correction (e.g., extrapolation) may be applied.

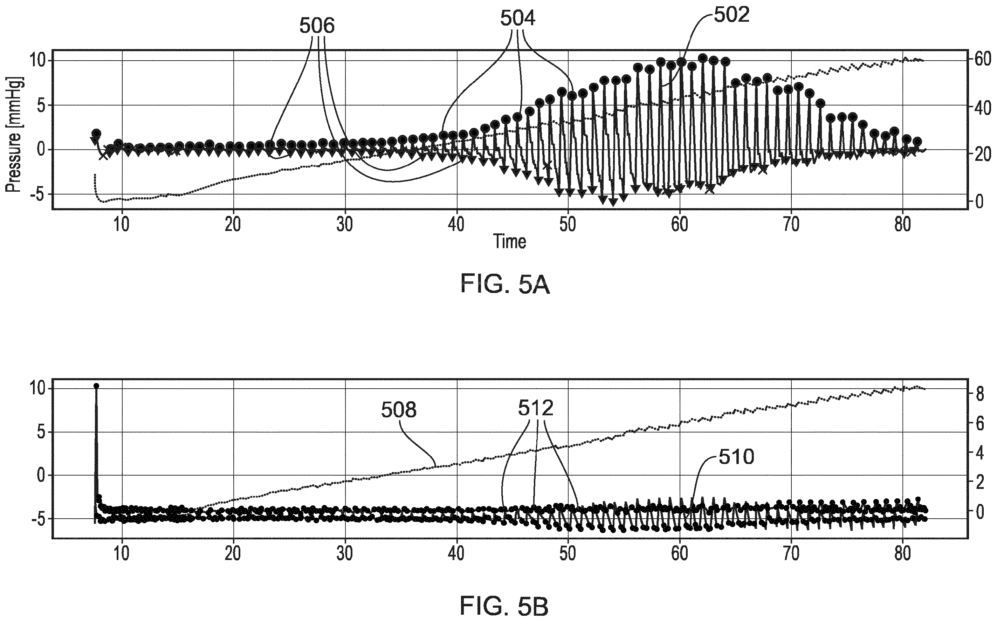

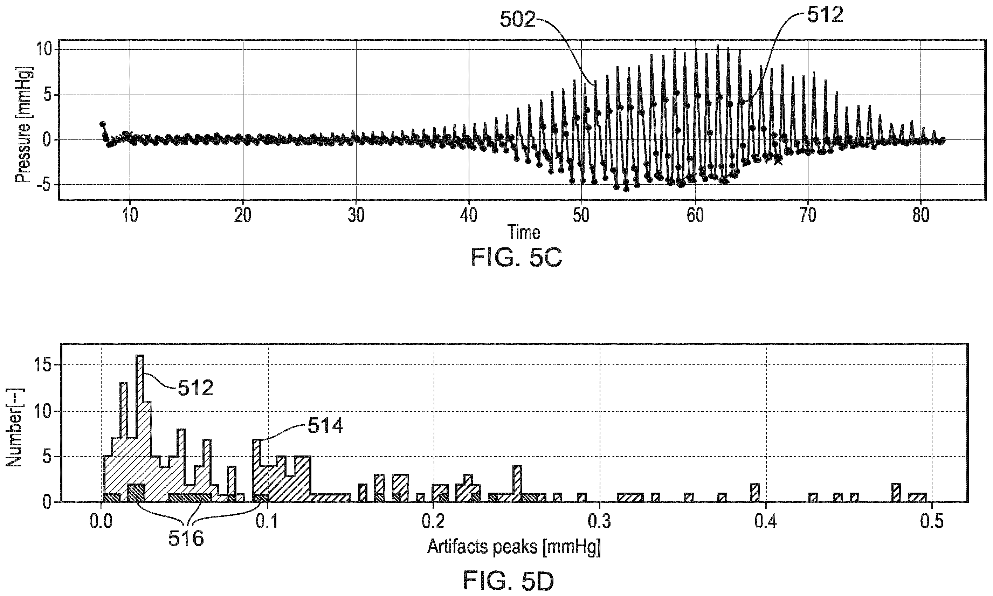

Figs. 5, 6, 7 and 8 show, graphically, data obtained from a blood pressure measurement sensor. In Fig. 5A, a line 502 represents tissue pressure (e.g., the pressure applied by the body part of the subject into the sensor pad 108) over time. In this example, the data represented by line 502 has been flattened, as discussed above. In Fig. 5A, circles 504 represent the pulse maxima and triangles 506 represent the pulse minima. In Fig. 5B, line 508 represents the applied pressure (which has been flattened), such as the pressure applied by the compression portion (e.g., an inflatable cuff) onto the body part of the subject. Line 510 represents the first derivative of the flattened applied pressure data. Dots 512 indicate points where the applied pressure is seen to suddenly drop. These drops are in this case global changes due to the control of components such as a pump used to inflate an inflatable cuff portion of the blood pressure measurement device, causing relatively small negative applied pressure gradients. In Fig. 5C, the dots 512 are plotted with the tissue data (line 502). Fig. 5D shows the number of artefacts detected in the data at each amplitude. The lines 514 show the total number of artefacts, and the lines 516 show the number of artefacts relating to stick-slip events. The data plotted in the graphs of Fig. 5 are based on real-life data, and it can be seen the number of stick-slip events is relatively small, and the amplitude of the artefacts relating to stick-slip events is also very small: less than 0.5 mmHg.

It is understood that the amplitude of artefacts detected in data is related to the difference between the static friction coefficient and the dynamic friction coefficient of the overlap region (Fig. 2; 202) of the shell (Fig. 1; 104). The larger the difference between the static friction coefficient and the dynamic friction coefficient, the larger the amplitude of the artefacts in the data (e.g., the larger the amplitude of the peaks in the applied pressure data). The data shown in Fig. 5 was obtained a blood pressure measurement device having a shell with a very small difference between the static friction coefficient and the dynamic friction coefficient (i.e., lower than 0.02).

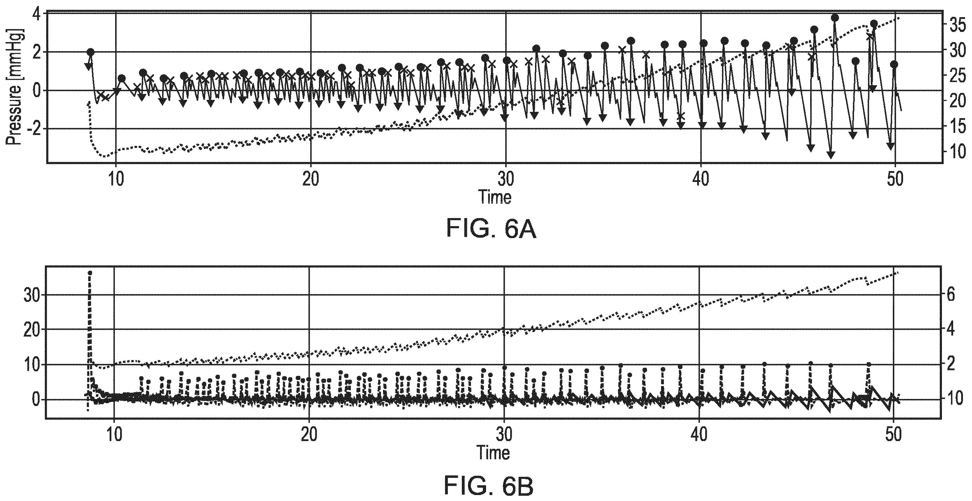

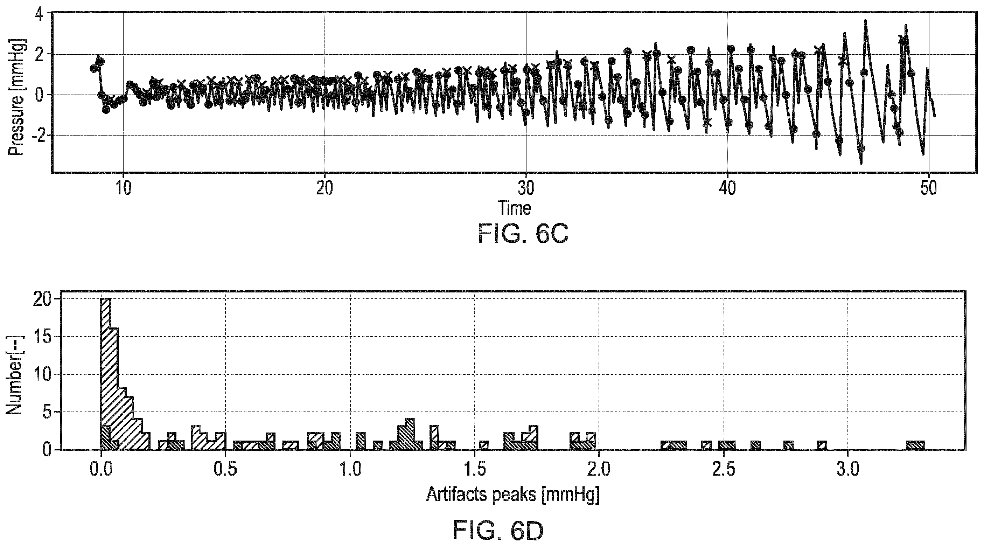

Fig. 6 shows graphs similar to those shown in Fig. 5, but the data plotted was obtained using a blood pressure measurement device having a shell with a much larger difference between the static friction coefficient and the dynamic friction coefficient (i.e., between 0.05 and 0.1). In Fig. 6A, far more peaks are evident between adjacent minima and maxima in the tissue pressure data. Once the first derivative of the applied pressure data has been determined (shown in Fig. 6B), and the sudden drops in the applied pressure data are plotted with the flattened tissue pressure data (shown in Fig. 6C), it becomes apparent that many more artefacts are detected, which correspond to stick-slip events, and which have high amplitudes (e.g., in the range of 0.3 to 12 mmHg). In this example, the artefacts may be considered to correspond to stick-slip events if they have an amplitude of between 0.3 mmHg to 15 mmHg or, more preferably, between 0.5 mmHg and 10 mmHg).

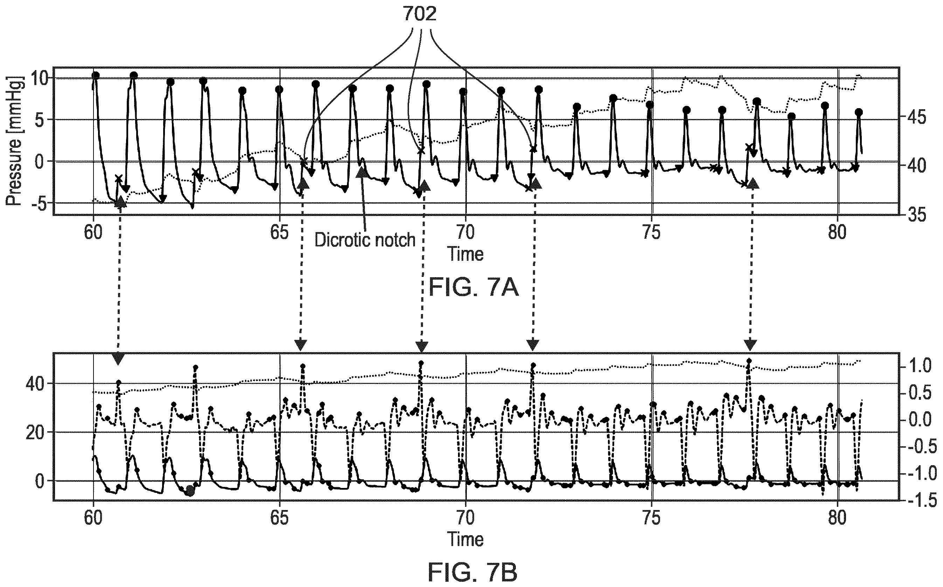

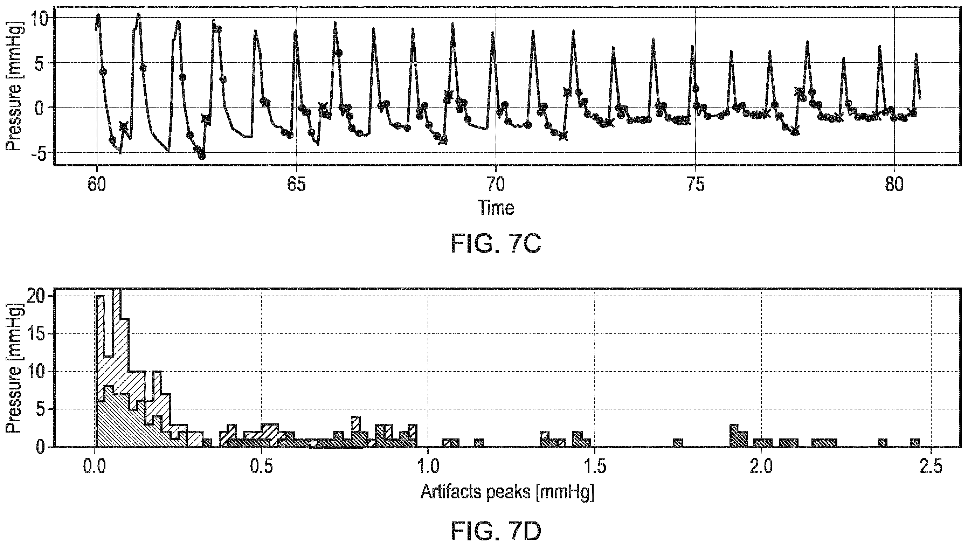

More example data is shown in the graphs of Fig. 7. In Fig. 7A, crosses 702 represent high-amplitude artefacts in the tissue pressure data that correspond to stick-slip events. Also indicated in Fig. 7 a is an occurrence of a peak in the tissue pressure data that is caused by the dicrotic notch, rather than a stick-slip event. Importantly, the present invention is able to determine that this peak does not result from a stick-slip event, and so the data is not ignored or corrected.

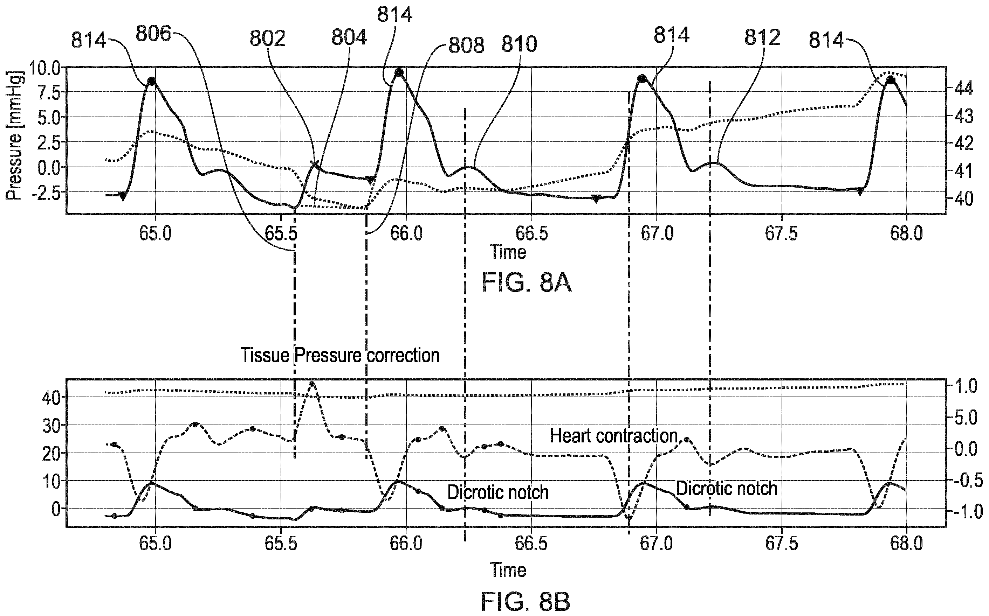

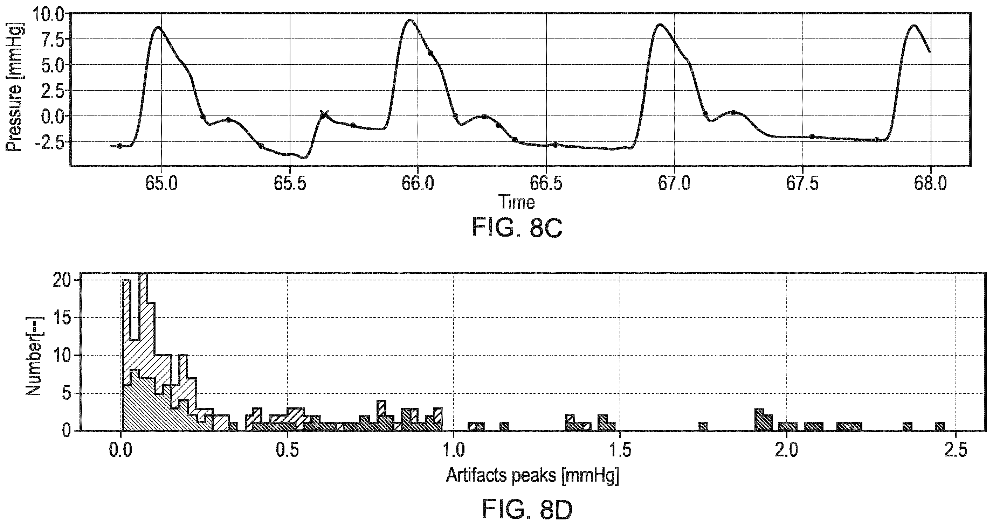

Fig. 8 shows some of the data from Fig. 7 over a shorter timescale. In Fig. 8A, a cross 802 indicates a peak corresponding to a stick-slip event. Dashed line 804 represents a correction made to the data using the interpolation techniques discussed above, extending from a first time 806, when the peak begins, to a second time 808, when the peak ends. The corrected data 804 removes the peak in the tissue pressure data caused by the stick-slip event. Figs. 8A and 7B also show peaks 810 and 812, which are determined to be caused by the dicrotic notch, and peaks 814 which are determined to be caused by a heart contraction.

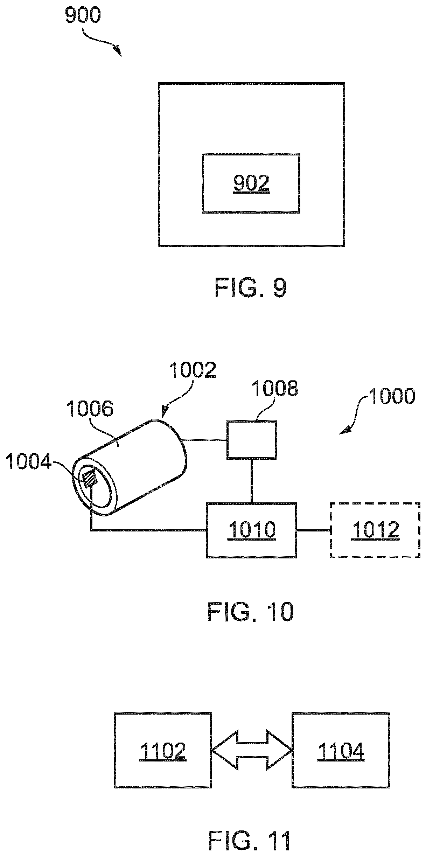

According to a second aspect, an apparatus is provided. Fig. 9 is a schematic illustration of an example of an apparatus 900 for detecting artefact in data acquired using a blood pressure measurement device. The apparatus 900 comprises a processor 902 configured to perform steps of the methods 300, 400 disclosed herein. The apparatus 900 may, in some embodiments, comprise a computing device.

According to a third aspect, a system is provided. Fig. 10 is a schematic illustration of an example of a system 1000 for detecting artefact in data acquired using a blood pressure measurement device. The system 1000 comprises a blood pressure measurement device 1002 which may, for example, comprise a blood pressure measurement cuff or some other device capable of measuring a blood pressure of a subject. The blood pressure measurement device 1002 (which may be the same as or similar to the blood pressure measurement device 100 discussed above) comprises a sensor pad 1004 (which may be the same as or similar to the sensor pad 108 discussed above) which is configured to measure a pressure applied on the sensor pad by tissue of a body part of a subject wearing a blood pressure measurement device. The blood pressure measurement device 1002 also comprises a compression portion 1006 configured to apply pressure to the body part of the subject. The compression portion 1006 may, for example, comprise an inflatable cuff portion (e.g., the inflatable cuff portion 106) or some other means for applying pressure, such as one or more straps or metal strips that can be tightened to apply pressure to the body part. The system 1000 further comprises an applied pressure sensor 1008 configured to measure the pressure applied to the body part of the subject by the compression portion 1006. By way of one example, the applied pressure sensor 1008 may be arranged to measure a pressure of gas inside an inflatable cuff portion 106. The system 1000 further comprises a processor 1010 (which may be the same as or similar to the processor 902 discussed above). The processor 1010 is configured to perform steps of the method 300, 400 discussed herein. Specifically, the processor 1010 is configured to receive, from the applied pressure sensor, applied pressure data indicative of the pressure applied to the body part of the subject; receive, from the sensor pad, tissue pressure data indicative of a pressure applied by the tissue of the body part of the subject on the sensor pad; determine the first derivative of the applied pressure data; identify an occurrence in the first derivative of the applied pressure data where an amplitude of the first derivative of the applied pressure data meets a first defined threshold condition; and responsive to determining that, within a defined duration of the occurrence, the tissue pressure meets a second defined threshold condition, determine that there exists an artefact in the tissue pressure data.

In some embodiments, the system 1000 may further comprise a user interface 1012 configured to present, to a recipient, a notification indicating the presence of the artefact in the tissue pressure data. In the event that multiple artefacts are detected (that exceed a threshold), the user interface 1012 may provide an indication to the recipient that the blood pressure measurement device should be checked for refitting or replacement. The apparatus 900 or the system 100 may comprise a health monitoring device or monitoring system, or a continuous patient monitoring device or monitoring system (e.g., a device or system in which data from multiple subjects are continuously monitored).

According to a fourth aspect, a computer program product is provided. Fig. 11 is a schematic illustration of an example of a computer-readable medium 1104 in communication with a processor 1102. The processor 102 may comprise or be similar to the processors 902 and 1010 discussed above. The computer program product comprises a non-transitory computer readable medium 1104, the computer-readable medium having computer-readable code embodied therein, the computer-readable code being configured such that, on execution by a suitable computer or processor 1102, the computer or processor is caused to perform steps of the method 300, 400 disclosed herein.

The processor 902, 1010, 1102 can comprise one or more processors, processing units, multi-core processors or modules that are configured or programmed to control the apparatus 900 in the manner described herein. In particular implementations, the processor 902, 1010, 1102 can comprise a plurality of software and/or hardware modules that are each configured to perform, or are for performing, individual or multiple steps of the method described herein.

The term "module", as used herein is intended to include a hardware component, such as a processor or a component of a processor configured to perform a particular function, or a software component, such as a set of instruction data that has a particular function when executed by a processor.

To further supplement understanding of the artefact detection principle discussed above, there will now be outlined in more detail a theoretical model which provides a means for more fine-grained analysis of potential artefacts and their causes.

In particular, this model allows for estimating whether a sudden increase in tissue pressure is due to an artefact, such as a stick-slip artefact, or caused by a hemodynamic event.

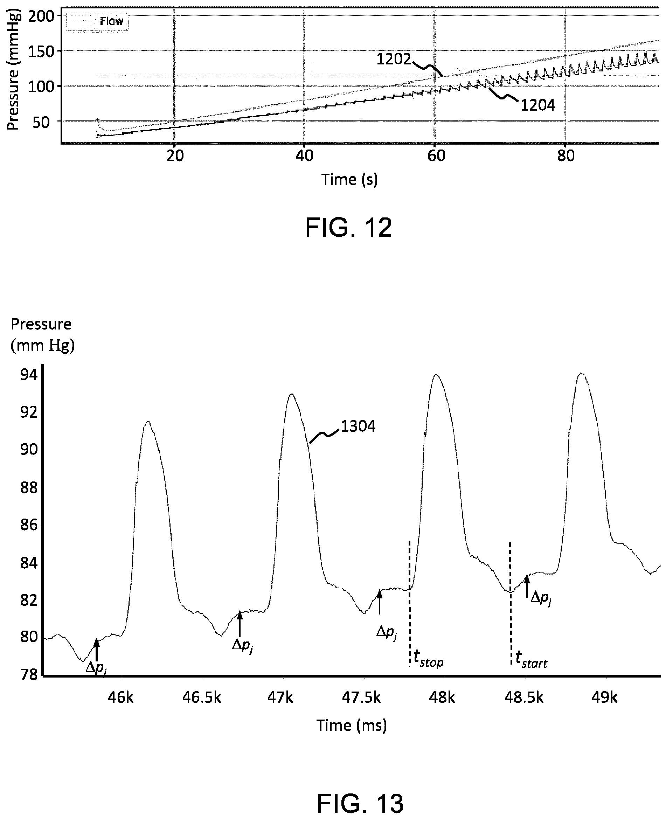

Fig. 12 shows an in-vivo recording of an applied pressure signal 1202 recorded by the applied pressure sensor and a tissue pressure signal 1204 recorded by the sensor pad 1004.

The applied pressure 1202 is linearly increasing as a function of time according to a certain slope $\alpha$. The tissue pressure signal 1204 recorded by the sensor pad 1004 is a combination of a linear increase in pressure, known as the clamping pressure (due to the increase in applied pressure) with an oscillating signal caused by the heart pulses, known as the tissue pressure oscillation.

The slope of the clamping pressure part of the sensor pad 1004 signal 1204 can in general be expected to be different from the slope, $\alpha$, of the applied pressure 1202, due mainly to friction of the overlapping shells of the device. The slope of the clamping pressure part of the sensor pad signal 1004 might be represented as $\beta = \varepsilon \cdot \alpha$, where $\varepsilon$ is factor < 1.

It is possible to determine a simple relation between the position of a potential stick-slip artefact in a heartbeat and the height of such a stick slip artefact.

Fig. 13 shows in-vivo recordings of the tissue sensor pad pressure signal 1304 of a test device which was engineered to be deliberately subject to stick-slip events, one during each heartbeat.

The stick-slip artefacts are clearly visible as sudden increases in the tissue pressure. The artefacts occur as a result of the slip phase of the stick-slip event. The start of each stick-slip artefact is an upward inflection in the signal. The end of each stick-slip artefact is marked by an arrow and a label, $\Delta p_j$, signifying the height of the resultant stick-slip artefact in the tissue pressure signal 1304, i.e. the pressure jump caused by the stick-slip event.

It is possible to relate the height of the pressure jump $\Delta p_j$ due to a stick-slip event to the location of the stick slip event relative to the preceding heartbeat.

From modelling, it is expected that the 'stick' phase of a stick-slip event will begin at the start of a heartbeat pulse. At this point, the overlapping parts 202 of the shell 104 of the cuff will stop sliding. This moment can be labelled $t_{stick}$. The 'slip' point of the stick-slip event, where the overlapping parts 202 of the shell 104 will start sliding again, can be labelled called $t_{slip}$. This would correspond to the start of the stick-slip artefact as it appears in the data signal.

When the overlapping parts of the shell 104 start sliding again, the clamping pressure part of the tissue pressure signal 1304 from the sensor pad 1004 will increase with an amplitude

$$\Delta p_{j,theory} \approx \beta \left( t_{slip} - t_{stick} \right)$$

where $\beta$ is the slope of the clamping pressure part of the sensor pad signal 1004, and can be determined empirically.

The slope, $\alpha$, of the applied pressure can be measured from the applied pressure sensor 1008. The slope $\beta$ can be determined directly from the clamping pressure part of the tissue pressure sensor signal 1304 from the sensor pad 1004, which is the trend line of the average increase in pressure of the tissue pressure signal measured by the sensor pad 1004 at the appropriate moment in time, i.e., where a suspected stick-slip event occurs. In other words, instead of computing the product $\beta = \varepsilon \cdot \alpha$, the slope $\beta$ can be determined directly from the tissue pressure signal from the sensor pad, for example by low-pass filtering the tissue pressure signal (to remove the arterial oscillation component) and computing the gradient of the resultant slope. The time point $t_{stick}$ can be determined from the positive inflection in the tissue pressure signal 1304 indicative of the foot of the blood pressure pulse immediately preceding the artefact. The time $t_{slip}$ can be determined from the moment of the pressure jump, i.e. the upward inflection in the signal not concurrent with the start of a blood pressure pulse, and concurrent with the start of the suspected stick-slip artefact.

Both $t_{slip}$ and $t_{stick}$ are indicated in Fig. 13.

In the illustrated case of Fig. 13, based on the above equation, it can be determined that $\Delta p_{j,theory} \approx 1.6$ mm Hg. This is indeed in good agreement with the indicated value from the figure ($\approx 1.5$ mmHg).

In accordance with one or more embodiments, it is proposed to use the above model to determine an expected magnitude of a suspected stick-slip artefact in the tissue pressure signal from the sensor pad 1004 as a function of the position of the artefact in the signal relative to at least one pulse wave in the signal, compare the expected magnitude of the artefact against the magnitude of the suspected artefact, and classify the suspected stick-slip artefact as a confirmed stick-slip artefact only if the true magnitude falls within a defined proximity range of the expected magnitude. It is noted that this procedure for determining whether a detected or suspected artefact in the tissue pressure signal corresponds to a stick-slip artefact can be applied to advantageous effect regardless of the particular method used to initially detect or identify the artifact or suspected artefact. In other words, this can be applied either in combination with any of the artifact detection method embodiments already discussed above or may be provided as a separate aspect of the invention.

The magnitude of the artefact may be defined as the height, $\Delta p_j$, of the artefact (in pressure), in the tissue pressure signal from the pressure sensor pad 1004.

The expected magnitude of the artefact may be computed using the model equation already set out above, based on determined time points $t_{slip}$ and $t_{stick}$ and based a determined value of the slope β.

In some embodiments, the suspected stick-slip artefact is classified as a confirmed stick-slip artefact only if the magnitude, $\Delta p_j$, of the suspected artefact in the tissue pressure signal from the sensor pad 1004, i.e. height of the artefact in the tissue pressure signal, meets the condition:

$$0.5\,\Delta p_{j,theory} \leq \Delta p_j \leq 1.5\Delta p_{j,theory}$$

where $\Delta p_{j,theory}$ is the expected magnitude (height) of the artefact, computed based on:

$$\Delta p_{j,theory} \approx \beta\left(t_{slip} - t_{stick}\right)$$

where the tissue pressure data includes a baseline slope component associated with the increasing applied pressure and an oscillation component associated with arterial pressure oscillations, and where $\beta$ is the gradient of the baseline slope component of the tissue pressure data, $t_{stick}$ is a time point of a start of a stick phase of the stick-slip artefact, and $t_{slip}$ is the time point of the start of a slip phase of the stick slip artefact, as discussed above. The time $t_{stick}$ can be determined to be the time point coinciding with the start or foot of the pulse wave signal immediately preceding the suspected artefact. The time $t_{slip}$ can be determined to be the time point coinciding with the start of the artefact.

As noted above, the procedure/method for determining/classifying whether an artefact or suspected artefact in the tissue pressure signal is a stick-slip artefact can be applied regardless of the particular method used to detect the artefact. To make this more concrete, there will be outlined below a recitation of a further aspect of the invention which may be provided alone or in combination with any of the features or embodiments already discussed above.

According to an aspect of the invention, there is provided a method for detecting/classifying a stick-slip artefact in data acquired using a blood pressure measurement device, the method comprising:

    receiving (304) tissue pressure data, the tissue pressure data indicative of a tissue pressure applied by the tissue of the subject on a sensor pad of the blood pressure measurement device;
    applying an artefact detection operation for detecting presence of an artefact in the tissue pressure data;
    responsive to determining that there exists an artefact in the tissue pressure data, determining whether the artefact is a stick-slip artifact.

In some embodiments, the determining whether the artefact is a stick-slip artifact comprises one or more of the following steps:

retrieving a pre-defined artefact model which models an expected magnitude of a stick-slip artefact in tissue pressure data received from a sensor pad as a function of a position of the artefact relative to at least one pulse wave in the tissue pressure data; determining a position of the detected artefact in the tissue pressure data relative to at least one pulse wave in the tissue pressure data and determining a true magnitude of the artefact in the tissue pressure data;
computing an expected magnitude of the artefact in the tissue pressure data if the artefact were a stick-slip artefact based on use of the model and the determined position of the detected artefact;
comparing the expected magnitude of the artefact with the true magnitude of the artefact;
classifying the artefact as a stick-slip artefact only if the true magnitude falls within a defined proximity range of the expected magnitude.

In some embodiments, the artefact model models an expected magnitude, $\Delta p_{j,theory}$, of a stick-slip artefact in tissue pressure data received from a sensor pad according to the equation:

$$\Delta p_{j,theory} \approx \beta\left(t_{slip} - t_{stick}\right)$$

where the tissue pressure data includes a baseline slope component associated with the increasing applied pressure and an oscillation component associated with arterial pressure oscillations, and where $\beta$ is the gradient of the baseline slope component of the tissue pressure data, $t_{stick}$ is a time point of a start of a stick phase of the stick-slip artefact, and $t_{slip}$ is the time point of the start of a slip phase of the stick slip artefact, and wherein $t_{stick}$ is defined as a time coinciding with a start point of a pulse wave in the tissue pressure data immediately preceding the artefact.

In some embodiments, the aforementioned artefact detection operation comprises determining whether the tissue pressure meets a defined threshold condition.

Additionally or alternatively, in some embodiments, the aforementioned artefact detection operation comprises:

    receiving applied pressure data, the applied pressure data comprising data indicative of a pressure applied to a body part of a subject whose blood pressure is to be measured;
    determining the first derivative of the applied pressure data; and
    identifying an occurrence in the first derivative of the applied pressure data where an amplitude of the first derivative of the applied pressure data meets a first defined threshold condition, and determining that there exists an artefact in the tissue pressure data responsive thereto.

Additionally or alternatively, in some embodiments, the aforementioned artefact detection operation comprises:

receiving applied pressure data, the applied pressure data comprising data indicative of a pressure applied to a body part of a subject whose blood pressure is to be measured;
determining the first derivative of the applied pressure data;
identifying an occurrence in the first derivative of the applied pressure data where an amplitude of the first derivative of the applied pressure data meets a first defined threshold condition; and
responsive to determining that, within a defined duration of the occurrence, the tissue pressure meets a second defined threshold condition, determining that there exists an artefact in the tissue pressure data.

Alternatively, any other method for detecting an artefact or suspected artefact in the tissue pressure data may instead be used. For example, this might comprise detecting a threshold slope of the tissue pressure signal, detecting a predefined waveform feature in the tissue pressure signal, or any other artefact detection approach.

Any embodiment of this aspect of the invention may be combined with any embodiment of feature outlined previously in relation to the other aspect(s) of the invention.

It will be appreciated that the embodiments of the invention also apply to computer programs, particularly computer programs on or in a carrier, adapted to put the invention into practice. The program may be in the form of a source code, an object code, a code intermediate source and an object code such as in a partially compiled form, or in any other form suitable for use in the implementation of the method according to embodiments of the invention. It will also be appreciated that such a program may have many different architectural designs. For example, a program code implementing the functionality of the method or system according to the invention may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g., Java interpreter instructions). Alternatively, one or more or all of the sub-routines may be stored in at least one external library file and linked with a main program either statically or dynamically, e.g., at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A computer-implemented method (300) for detecting an artefact in data acquired using a blood pressure measurement device, the method comprising:

receiving (302) applied pressure data, the applied pressure data comprising data indicative of a pressure applied to a body part of a subject whose blood pressure is to be measured;

receiving (304) tissue pressure data, the tissue pressure data indicative of a tissue pressure applied by the tissue of the subject on a sensor pad of the blood pressure measurement device; determining (306) the first derivative of the applied pressure data; identifying (308) an occurrence in the first derivative of the applied pressure data where an amplitude of the first derivative of the applied pressure data meets a first defined threshold condition; and responsive to determining that, within a defined duration of the occurrence, the tissue pressure meets a second defined threshold condition, determining (310) that there exists an artefact in the tissue pressure data.

2. A computer-implemented method (300, 400) according to claim 1, further comprising: generating (406), for delivery to a recipient device, a notification of the presence of the artefact in the tissue pressure data.

3. A computer-implemented method (300, 400) according to claim 1 or claim 2, further comprising:

   determining (410) a correction to be applied to the tissue pressure data to remove the artefact; and applying (412) the correction to the tissue pressure data.

4. A computer-implemented method (300, 400) according to claim 3, wherein determining the correction comprises interpolating the first derivative of the tissue pressure data over a defined interpolation time period.

5. A computer-implemented method (300, 400) according to any of the preceding claims, further comprising: responsive to determining that the tissue pressure meets the second defined threshold condition not within the defined duration of the occurrence, determining (404) that the tissue pressure data includes an event indicative of a hemodynamic effect.

6. A computer-implemented method (300, 400) according to claim 5, wherein the hemodynamic effect comprises at least one of a dicrotic notch event and a heart contractility event.

7. A computer-implemented method (300, 400) according to any of the preceding claims, further comprising: responsive to determining that the artefact is one of a plurality of artefacts identified in the tissue pressure data, and that the number of artefacts in the plurality of artefacts exceeds a defined threshold number, generating (408), for delivery to a recipient device, a notification that action should be taken with regard to the blood pressure measurement device.

8. A computer-implemented method (300, 400) according to any of the preceding claims, further comprising: applying (402) a data flattening operation to at least one of the applied pressure data and the tissue pressure data.

9. A computer-implemented method (300, 400) according to claim 8, wherein determining the first derivative of the applied pressure data comprises determining the negative first derivative of the applied pressure data; and wherein identifying an occurrence where an amplitude of the first derivative of the applied pressure data meets a first defined threshold condition comprises identifying an occurrence where the amplitude of the first derivative is greater than 0.

10. A computer-implemented method (300, 400) according to any of the preceding claims, wherein the defined duration comprises a duration of 100 ms before or after the occurrence.

11. A computer-implemented method (300, 400) according to any of the preceding claims, wherein the second defined threshold condition is met when the amplitude of the tissue pressure increases above a defined threshold pressure within the defined duration of the occurrence, the defined threshold pressure being between 0.05 and 50 mmHg.

12. A computer-implemented method (300, 400) according to any of the preceding claims, wherein the method further comprises, following determining (310) that there exists an artefact in the tissue pressure data, determining whether the artefact is a stick-slip artifact, wherein the determining comprises:

    retrieving a pre-defined artefact model which models an expected magnitude of a stick-slip artefact in tissue pressure data received from a sensor pad as a function of a position of the artefact relative to at least one pulse wave in the tissue pressure data; determining a position of the detected artefact in the tissue pressure data relative to at least one pulse wave in the tissue pressure data and determining a true magnitude of the artefact in the tissue pressure data; computing an expected magnitude of the artefact in the tissue pressure data if the artefact were a stick-slip artefact based on use of the model and the determined position of the detected artefact;
comparing the expected magnitude of the artefact with the true magnitude of the artefact;
classifying the artefact as a stick-slip artefact only if the true magnitude falls within a defined proximity range of the expected magnitude.

13. A computer implemented method (300, 400) according to claim 12, wherein the artefact model models an expected magnitude, $\Delta p_{j,theory}$, of a stick-slip artefact in tissue pressure data received from a sensor pad according to the equation:

$$\Delta p_{j,theory} \approx \beta\left(t_{slip} - t_{stick}\right)$$

where the tissue pressure data includes a baseline slope component associated with the increasing applied pressure and an oscillation component associated with arterial pressure oscillations, and where $\beta$ is the gradient of the baseline slope component of the tissue pressure data, $t_{stick}$ is a time point of a start of a stick phase of the stick-slip artefact, and $t_{slip}$ is the time point of the start of a slip phase of the stick slip artefact, and wherein $t_{stick}$ is defined as a time coinciding with a start point of a pulse wave in the tissue pressure data immediately preceding the artefact.

14. An apparatus (900) for detecting an artefact in data acquired using a blood pressure measurement device, the apparatus comprising:
a processor (902) configured to perform the method according to any of the preceding claims.

15. A system (1000) for detecting an artefact in data acquired using a blood pressure measurement device, the system comprising:
a blood pressure measurement device (1002) comprising:

a sensor pad (1004) configured to measure a pressure applied on the sensor pad by tissue of a body part of a subject wearing the blood pressure measurement device; and
a compression portion (1006) configured to apply pressure to the body part of the subject;
an applied pressure sensor (1008) configured to measure the pressure applied to the body part of the subject by the compression portion; and
a processor (1010) configured to:

receive, from the applied pressure sensor, applied pressure data indicative of the pressure applied to the body part of the subject;
receive, from the sensor pad, tissue pressure data indicative of a pressure applied by the tissue of the body part of the subject on the sensor pad;
determine the first derivative of the applied pressure data;
identify an occurrence in the first derivative of the applied pressure data where an amplitude of the first derivative of the applied pressure data meets a first defined threshold condition; and
responsive to determining that, within a defined duration of the occurrence, the tissue pressure meets a second defined threshold condition, determine that there exists an artefact in the tissue pressure data.

16. A system (1000) according to claim 15, further comprising:
a user interface (1012) configured to present, to a recipient, a notification indicating the presence of the artefact in the tissue pressure data.

17. A computer program product comprising a non-transitory computer-readable medium (1104), the computer-readable medium having computer-readable code embodied therein, the computer-readable code being configured such that, on execution by a suitable computer or processor, the computer or processor (1102) is caused to perform the method of any of claims 1 to 13.

**Patentansprüche**

1. Computerimplementiertes Verfahren (300) zum Detektieren eines Artefakts in Daten, die mit einer Blutdruckmessvorrichtung erfasst wurden, wobei das Verfahren Folgendes umfasst:

Empfangen (302) von Daten über einen ausgeübten Druck, wobei die Daten über einen ausgeübten Druck Daten umfassen, die einen auf einen Körperteil eines Subjekts ausgeübten Druck angeben, dessen Blutdruck gemessen werden soll;
Empfangen (304) von Gewebedruckdaten, wobei die Gewebedruckdaten einen Gewebedruck angeben, der vom Gewebe des Subjekts auf ein Sensorfeld der Blutdruckmessvorrichtung ausgeübt wird;
Bestimmen (306) der ersten Ableitung der Daten über einen ausgeübten Druck;
Identifizieren (308) eines Auftretens in der ersten Ableitung der Daten über einen ausgeübten Druck, bei dem eine Amplitude der ersten Ableitung der Daten über einen ausgeübten Druck eine erste definierte Schwellenbedingung erfüllt; und
infolge eines Bestimmens, dass der Gewebedruck innerhalb einer definierten Dauer des Auftretens eine zweite definierte Schwellenbedingung erfüllt, Bestimmen (310), dass ein Artefakt in den Gewebedruckdaten vorliegt.

2. Computerimplementiertes Verfahren (300, 400) nach Anspruch 1, das weiter Folgendes umfasst: Erzeugen (406), zur Übermittlung an eine Empfangsvorrichtung, einer Benachrichtigung über das Vorhandensein des Artefakts in den Gewebedruckdaten.

3. Computerimplementiertes Verfahren (300, 400) nach Anspruch 1 oder Anspruch 2, das weiter Folgendes umfasst:

   Bestimmen (410) einer Korrektur, die auf die Gewebedruckdaten angewendet werden soll, um das Artefakt zu entfernen; und Anwenden (412) der Korrektur auf die Gewebedruckdaten.

4. Computerimplementiertes Verfahren (300, 400) nach Anspruch 3, wobei Bestimmen der Korrektur Interpolieren der ersten Ableitung der Gewebedruckdaten über einen definierten Interpolationszeitraum umfasst.

5. Computerimplementiertes Verfahren (300, 400) nach einem der vorstehenden Ansprüche, das weiter Folgendes umfasst: infolge eines Bestimmens, dass der Gewebedruck die zweite definierte Schwellenbedingung nicht innerhalb der definierten Dauer des Auftretens erfüllt, Bestimmen (404), dass die Gewebedruckdaten ein Ereignis beinhalten, das einen hämodynamischen Effekt angibt.

6. Computerimplementiertes Verfahren (300, 400) nach Anspruch 5, wobei der hämodynamische Effekt mindestens eines von einem dikrotischen Kerbereignis und einem Herzkontraktilitätsereignis umfasst.

7. Computerimplementiertes Verfahren (300, 400) nach einem der vorstehenden Ansprüche, das weiter Folgendes umfasst: infolge eines Bestimmens, dass das Artefakt eines von einer Vielzahl von in den Gewebedruckdaten identifizierten Artefakten ist, und dass die Anzahl von Artefakten in der Vielzahl von Artefakten einen definierten Schwellenwert überschreitet, Erzeugen (408), zur Übermittlung an eine Empfangsvorrichtung, einer Benachrichtigung, dass in Bezug auf die Blutdruckmessvorrichtung eine Maßnahme ergriffen werden sollte.

8. Computerimplementiertes Verfahren (300, 400) nach einem der vorstehenden Ansprüche, das weiter Folgendes umfasst:
Anwenden (402) einer Datenglättungsoperation auf mindestens eine der Daten über einen ausgeübten Druck und die Gewebedruckdaten.

9. Computerimplementiertes Verfahren (300, 400) nach Anspruch 8, wobei Bestimmen der ersten Ableitung der Daten über einen ausgeübten Druck Bestimmen der negativen ersten Ableitung der Daten über einen ausgeübten Druck umfasst; und wobei Identifizieren eines Auftretens, bei dem eine Amplitude der ersten Ableitung der Daten über einen ausgeübten Druck eine erste definierte Schwellenbedingung erfüllt, Identifizieren eines Auftretens umfasst, bei dem die Amplitude der ersten Ableitung größer als 0 ist.

10. Computerimplementiertes Verfahren (300, 400) nach einem der vorstehenden Ansprüche, wobei die definierte Dauer eine Dauer von 100 ms vor oder nach dem Auftreten umfasst.

11. Computerimplementiertes Verfahren (300, 400) nach einem der vorstehenden Ansprüche, wobei die zweite definierte Schwellenbedingung erfüllt ist, wenn die Amplitude des Gewebedrucks innerhalb der definierten Dauer des Auftretens über einen definierten Schwellendruck steigt, wobei der definierte Schwellendruck zwischen 0,05 und 50 mmHg liegt.

12. Computerimplementiertes Verfahren (300, 400) nach einem der vorstehenden Ansprüche, wobei das Verfahren weiter, nach Bestimmen (310), dass in den Gewebedruckdaten ein Artefakt existiert, Bestimmen umfasst, ob es sich bei dem Artefakt um ein Haftgleitartefakt handelt, wobei das Bestimmen Folgendes umfasst:

   Abrufen eines vordefinierten Artefaktmodells, das eine erwartete Größe eines Haftgleitartefakts in Gewebedruckdaten, die von einem Sensorfeld empfangen werden, als eine Funktion einer Position des Artefakts relativ zu mindestens einer Pulswelle in den Gewebedruckdaten modelliert;
   Bestimmen einer Position des detektierten Artefakts in den Gewebedruckdaten relativ zu mindestens einer Pulswelle in den Gewebedruckdaten und Bestimmen einer tatsächlichen Größe des Artefakts in den Gewebedruckdaten;
   Berechnen einer zu erwartenden Stärke des Artefakts in den Gewebedruckdaten, falls es sich bei dem Artefakt um ein Haftgleitartefakt handeln würde, basierend auf der Verwendung des Modells und der bestimmten Position des detektierten Artefakts;
   Vergleichen der erwarteten Größe des Artefakts mit der tatsächlichen Größe des Artefakts; Klassifizieren des Artefakts als ein Haftgleitartefakt nur dann, wenn die tatsächliche Größe innerhalb eines definierten Nahbereichs der erwarteten Größe liegt.

13. Computerimplementiertes Verfahren (300, 400) nach Anspruch 12, wobei das Artefaktmodell eine erwartete Größe, $\Delta p_{j, Theorie}$, eines Haftgleitartefakts in Gewebedruckdaten, die von einem Sensorfeld empfangen werden, gemäß der folgenden Gleichung modelliert:

$$\Delta p_{j,Theorie} \approx \beta(t_{Gleiten} - t_{Haften})$$

wobei die Gewebedruckdaten eine Basisliniensteigungskomponente, die mit dem zunehmenden ausgeübten Druck verknüpft ist, und eine Oszillationskomponente, die mit arteriellen Druckoszillationen verknüpft ist, beinhalten und wobei β der Gradient der Basisliniensteigungskomponente der Gewebedruckdaten ist, $t_{Haften}$ ein Zeitpunkt eines Starts einer Haftphase des Haftgleitartefakts ist und $t_{Gleiten}$ der Zeitpunkt des Starts einer Gleitphase des Haftgleitartefakts ist, und wobei $t_{Haften}$ als ein Zeitpunkt definiert ist, der mit einem Startpunkt einer Pulswelle in den Gewebedruckdaten unmittelbar vor dem Artefakt übereinstimmt.

14. Einrichtung (900) zum Detektieren eines Artefakts in Daten, die mit einer Blutdruckmessvorrichtung erfasst wurden, wobei die Einrichtung Folgendes umfasst:
einen Prozessor (902), der dazu konfiguriert ist, das Verfahren nach einem der vorstehenden Ansprüche durchzuführen.

15. System (1000) zum Detektieren eines Artefakts in Daten, die mit einer Blutdruckmessvorrichtung erfasst wurden, wobei das System Folgendes umfasst:
eine Blutdruckmessvorrichtung (1002), umfassend:

ein Sensorfeld (1004), das dazu konfiguriert ist, den Druck zu messen, der von Gewebe eines Körperteils eines Subjekts, das die Blutdruckmessvorrichtung trägt, auf das Sensorfeld ausgeübt wird; und
einen Kompressionsabschnitt (1006), der dazu konfiguriert ist, Druck auf den Körperteil des Subjekts auszuüben;
ein Sensor für ausgeübten Druck (1008), der dazu konfiguriert ist, den Druck zu messen, der durch den Kompressionsabschnitt auf den Körperteil des Subjekts ausgeübt wird; und
einen Prozessor (1010), der für Folgendes konfiguriert ist:

Empfangen, vom Sensor für ausgeübten Druck, von Daten über einen ausgeübten Druck, die den Druck angeben, der vom Körperteil des Subjekts ausgeübt wird;
Empfangen, vom Sensorfeld, von Gewebedruckdaten, die einen Druck angeben, der vom Gewebe des Körperteils des Subjekts auf das Sensorfeld ausgeübt wird;
Bestimmen der ersten Ableitung der Daten über einen ausgeübten Druck;
Identifizieren eines Auftretens in der ersten Ableitung der Daten über einen ausgeübten Druck, bei dem eine Amplitude der ersten Ableitung der Daten über einen ausgeübten Druck eine erste definierte Schwellenbedingung erfüllt; und
infolge eines Bestimmens, dass der Gewebedruck innerhalb einer definierten Dauer des Auftretens eine zweite definierte Schwellenbedingung erfüllt, Bestimmen, dass ein Artefakt in den Gewebedruckdaten vorliegt.

16. System (1000) nach Anspruch 15, das weiter Folgendes umfasst:
eine Benutzeroberfläche (1012), die dazu konfiguriert ist, einem Empfänger eine Benachrichtigung darzustellen, die das Vorhandensein des Artefakts in den Gewebedruckdaten angibt.

17. Computerprogrammprodukt, das ein nichtflüchtiges computerlesbares Medium (1104) umfasst, wobei das computerlesbare Medium computerlesbaren Code darin enthalten aufweist, wobei der computerlesbare Code so konfiguriert ist, dass bei Ausführung durch einen geeigneten Computer oder Prozessor, der Computer oder Prozessor (1102) veranlasst wird, das Verfahren nach einem der Ansprüche 1 bis 13 durchzuführen.

**Revendications**

1. Procédé mis en œuvre par ordinateur (300) de détection d'un artéfact dans des données acquises en utilisant un dispositif de mesure de pression sanguine, le procédé comprenant :

la réception (302) de données de pression appliquée, les données de pression appliquée comprenant des données indiquant une pression appliquée à une partie du corps d'un sujet dont la pression sanguine doit être mesurée ;
la réception (304) de données de pression de tissu, les données de pression de tissu indiquant une pression de tissu appliquée par le tissu du sujet sur une pastille de capteur du dispositif de mesure de pression sanguine ;

la détermination (306) de la première dérivée des données de pression appliquée ;

l'identification (308) d'une occurrence dans la première dérivée des données de pression appliquée où une amplitude de la première dérivée des données de pression appliquée satisfait une première condition de seuil définie ; et

en réponse à la détermination que, au sein d'une durée définie de l'occurrence, la pression de tissu satisfait une seconde condition de seuil définie, la détermination (310) qu'il existe un artéfact dans les données de pression de tissu.

2. Procédé mis en œuvre par ordinateur (300, 400) selon la revendication 1, comprenant en outre :
la génération (406), pour livraison à un dispositif destinataire, d'une notification de la présence de l'artéfact dans les données de pression de tissu.

3. Procédé mis en œuvre par ordinateur (300, 400) selon la revendication 1 ou 2, comprenant en outre :

la détermination (410) d'une correction à appliquer aux données de pression de tissu pour retirer l'artéfact ; et
l'application (412) de la correction aux données de pression de tissu.

4. Procédé mis en œuvre par ordinateur (300, 400) selon la revendication 3, dans lequel la détermination de la correction comprend l'interpolation de la première dérivée des données de pression de tissu sur une période de temps d'interpolation définie.

5. Procédé mis en œuvre par ordinateur (300, 400) selon l'une quelconque des revendications précédentes, comprenant en outre :
en réponse à la détermination que la pression de tissu répond à la seconde condition de seuil définie pas au sein de la durée définie de l'occurrence, la détermination (404) que les données de pression de tissu incluent un événement indiquant un effet hémodynamique.

6. Procédé mis en œuvre par ordinateur (300, 400) selon la revendication 5, dans lequel l'effet hémodynamique comprend au moins un parmi un événement d'encoche dicrote et un événement de contractilité cardiaque.

7. Procédé mis en œuvre par ordinateur (300, 400) selon l'une quelconque des revendications précédentes, comprenant en outre :
en réponse à la détermination que l'artéfact fait partie d'une pluralité d'artéfacts identifiés dans les données de pression de tissu, et que le nombre d'artéfacts dans la pluralité d'artéfacts dépasse un nombre seuil défini, la génération (408), pour livraison à un dispositif destinataire, d'une notification qu'une mesure doit être prise concernant le dispositif de mesure de pression sanguine.

8. Procédé mis en œuvre par ordinateur (300, 400) selon l'une quelconque des revendications précédentes, comprenant en outre :
l'application (402) d'une opération d'aplatissement de données à au moins une parmi les données de pression appliquée et données de pression de tissu.

9. Procédé mis en œuvre par ordinateur (300, 400) selon la revendication 8, dans lequel la détermination de la première dérivée des données de pression appliquée comprend la détermination de la première dérivée négative des données de pression appliquée ; et
dans lequel l'identification d'une occurrence où une amplitude de la première dérivée des données de pression appliquée satisfait une première condition de seuil définie comprend l'identification d'une occurrence où l'amplitude de la première dérivée est supérieure à 0.

10. Procédé mis en œuvre par ordinateur (300, 400) selon l'une quelconque des revendications précédentes, dans lequel la durée définie comprend une durée de 100 ms avant ou après l'occurrence.

11. Procédé mis en œuvre par ordinateur (300, 400) selon l'une quelconque des revendications précédentes, dans lequel la seconde condition de seuil définie est atteinte lorsque l'amplitude de la pression de tissu augmente au-dessus d'une pression de seuil définie au sein de la durée définie de l'occurrence, la pression de seuil définie étant entre 0,05 et 50 mmHg.

12. Procédé mis en œuvre par ordinateur (300, 400) selon l'une quelconque des revendications précédentes, dans lequel le procédé comprend en outre, après la détermination (310) qu'il existe un artéfact dans les données de pression de tissu, la détermination si l'artéfact est un artéfact de stick-slip, dans lequel la détermination comprend :

la récupération d'un modèle d'artéfact prédéfini qui modélise une magnitude attendue d'un artéfact de stick-slip dans des données de pression de tissu reçues à partir d'une pastille de capteur en fonction d'une position de l'artéfact par rapport à au moins une onde de pouls dans les données de pression de tissu ;
la détermination d'une position de l'artéfact détecté dans les données de pression de tissu par rapport à au moins une onde de pouls dans les données de pression de tissu et la détermination d'une magnitude réelle de l'artéfact dans les données de pression de tissu ;

le calcul d'une magnitude attendue de l'artéfact dans les données de pression de tissu si l'artéfact était un artéfact de stick-slip sur la base de l'utilisation du modèle et de la position déterminée de l'artéfact détecté ;

la comparaison de la magnitude attendue de l'artéfact avec la magnitude réelle de l'artéfact ;

le classement de l'artéfact comme un artéfact de stick-slip uniquement si la magnitude réelle tombe au sein d'une plage de proximité définie de la magnitude attendue.

13. Procédé mis en œuvre par ordinateur (300, 400) selon la revendication 12, dans lequel le modèle d'artéfact modélise une magnitude attendue, $\Delta p_{j,theory}$, d'un artéfact de stick-slip dans les données de pression de tissu reçues à partir d'une pastille de capteur selon l'équation :

$$\Delta p_{j,theory} \approx \beta(t_{slip} - t_{stick})$$

où les données de pression de tissu incluent une composante de pente de base associée à la pression appliquée en hausse et une composante d'oscillation associée à des oscillations de pression sanguine, et où $\beta$ est le gradient de la composante de pente de base des données de pression de tissu, $t_{stick}$ est un instant d'un début d'une phase de stick de l'artéfact de stick-slip, et $t_{slip}$ est l'instant du début d'une phase de slip de l'artéfact de stick-slip, et dans lequel $t_{stick}$ est défini comme un temps coïncidant avec un point de départ d'une onde de pouls dans les données de pression de tissu précédant immédiatement l'artéfact.

14. Appareil (900) pour détecter un artéfact dans des données acquises en utilisant un dispositif de mesure de pression sanguine, l'appareil comprenant :
un processeur (902) configuré pour réaliser le procédé selon l'une quelconque des revendications précédentes.

15. Système (1000) pour détecter un artéfact dans des données acquises en utilisant un dispositif de mesure de pression sanguine, le système comprenant :
un dispositif de mesure de pression sanguine (1002) comprenant :

une pastille de capteur (1004) configurée pour mesurer une pression appliquée sur la pastille de capteur par du tissu d'une partie du corps d'un sujet portant le dispositif de mesure de pression sanguine ; et
une portion de compression (1006) configurée pour appliquer de la pression sur la partie du corps du sujet ;

un capteur de pression appliquée (1008) configuré pour mesurer la pression appliquée à la partie du corps du sujet par la portion de compression ; et
un processeur (1010) configuré pour :

recevoir, à partir du capteur de pression appliqué, des données de pression appliquée indiquant la pression appliquée à la partie du corps du sujet ;
recevoir, à partir de la pastille de capteur, des données de pression de tissu indiquant une pression appliquée par le tissu de la partie du corps du sujet sur la pastille de capteur ;
déterminer la première dérivée des données de pression appliqué ;
identifier une occurrence dans la première dérivée des données de pression appliquée où une amplitude de la première dérivée des données de pression appliquée satisfait une première condition de seuil définie ; et
en réponse à la détermination que, au sein d'une durée définie de l'occurrence, la pression de tissu satisfait une seconde condition de seuil définie, déterminer qu'il existe un artéfact dans les données de pression de tissu.

16. Système (1000) selon la revendication 15, comprenant en outre :
une interface utilisateur (1012) configurée pour présenter, à un destinataire, une notification indiquant la présence de l'artéfact dans les données de pression de tissu.

17. Produit de programme informatique comprenant un support lisible par ordinateur non transitoire (1104), le support lisible par ordinateur étant configuré de sorte que, à l'exécution par un ordinateur ou un processeur adapté, l'ordinateur ou le processeur (1102) est amené à mettre en œuvre le procédé selon l'une quelconque des revendications 1 à 13.

FIG. 1

FIG. 2

300

302

304

306

308

310

FIG. 3

400

302

304

402

306

308 → 404

310 → 408

406

410

412

FIG. 4

FIG. 5A

FIG. 5B

FIG. 5C

FIG. 5D

FIG. 6A

FIG. 6B

FIG. 6C

FIG. 6D

FIG. 7A

FIG. 7B

FIG. 7C

FIG. 7D

EP 4 637 533 B1

FIG. 8A

FIG. 8B

EP 4 637 533 B1

FIG. 8C

FIG. 8D

900

902

FIG. 9

1002
1006
1008
1000
1004

1010

1012

FIG. 10

1102

1104

FIG. 11

FIG. 12

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2012245478 A1
- US 2021121073 A1
- US 8998817 B
- EP 2953528 A
- EP 2022075999 W